# EUROPÄISCHE PATENTSCHRIFT

(11) **EP 1 398 321 B1**
(45) Veröffentlichungstag und Bekanntmachung des Hinweises auf die Patenterteilung: **05.04.2006**
(21) Anmeldenummer: 03020183.4
(22) Anmeldetag: 05.09.2003
(51) Int. Cl.: C07K 14/415, G01N 33/68

(54) **Forisomen, Verfahren zu ihrer Isolierung und ihre Verwendung als molekulare Arbeitsmaschine**
Forisomes, process for their preparation and their use as molecular machine
Forisomes, procédé pour leur préparation et leur emploi en tant que machine moléculaire

(30) Priorität: 09.09.2002 DE 10241681
(43) Veröffentlichungstag der Anmeldung: 17.03.2004
(73) Patentinhaber: FRAUNHOFER-GESELLSCHAFT ZUR FÖRDERUNG DER ANGEWANDTEN FORSCHUNG E.V., 80686 München (DE)
(72) Erfinder: Knoblauch, Michael, Dr., 35510 Butzbach (DE); Prüfer, Dirk, Dr., 50939 Köln (DE)

(56) Entgegenhaltungen:
- DATABASE CA [Online] CHEMICAL ABSTRACTS SERVICE, COLUMBUS, OHIO, US; KNOBLAUCH, MICHAEL ET AL: "ATP-independent contractile proteins from plants" retrieved from STN Database accession no. 140:2831 CA XP002266445 & NATURE MATERIALS (2003), 2(9), 600-603 , 2003,
- DATABASE CA [Online] CHEMICAL ABSTRACTS SERVICE, COLUMBUS, OHIO, US; BEHNKE, H. -D.: "Sieve-element plastids, nuclear crystals and phloem proteins in the Zingiberales" retrieved from STN Database accession no. 121:78319 CA XP002266446 & BOTANICA ACTA (1994), 107(1), 3-11 , 1994,
- DATABASE CA [Online] CHEMICAL ABSTRACTS SERVICE, COLUMBUS, OHIO, US; ARSANTO, JEAN PIERRE: "Observations on P-protein in dicotyledons. Substructural and developmental features" retrieved from STN Database accession no. 97:123998 CA XP002266447 & AMERICAN JOURNAL OF BOTANY (1982), 69(7), 1200-12 , 1982,
- DATABASE CA [Online] CHEMICAL ABSTRACTS SERVICE, COLUMBUS, OHIO, US; KNOBLAUCH, MICHAEL ET AL: "Reversible calcium-regulated stopcocks in legume sieve tubes" retrieved from STN Database accession no. 135:150071 CA XP002266448 & PLANT CELL (2001), 13(5), 1221-1230 , 2001,
- D BHATTACHARYA ET AL.: "Molecular cloning and evolutionary analysis of the calcium-modulated contractile protein, centrin, in green algae and land plants" PLANT MOLECULAR BIOLOGY., Bd. 23, Nr. 1, 1993, Seiten 1243-1254, XP002266444 NIJHOFF PUBLISHERS, DORDRECHT., NL ISSN: 0167-4412

## Beschreibung

Die vorliegende Erfindung betrifft Forisomen, d.h. Proteinkörper, die auch als kristalline P-Proteine bezeichnet werden und nach bisherigem Kenntnisstand ausschließlich, aber dort ubiquitär in Pflanzen aus der Familie der Fabaceen (Fabaceae, Papillionaceae) vorkommen. Diese Proteinkörper besitzen ganz außerordentliche, bisher nicht bekannte Eigenschaften, die es ermöglichen, sie als molekulare Arbeitsmaschinen zu verwenden. Weiterhin betrifft die Erfindung ein Verfahren, wie sich Forisomen aus ihrer natürlichen Umgebung isolieren lassen.

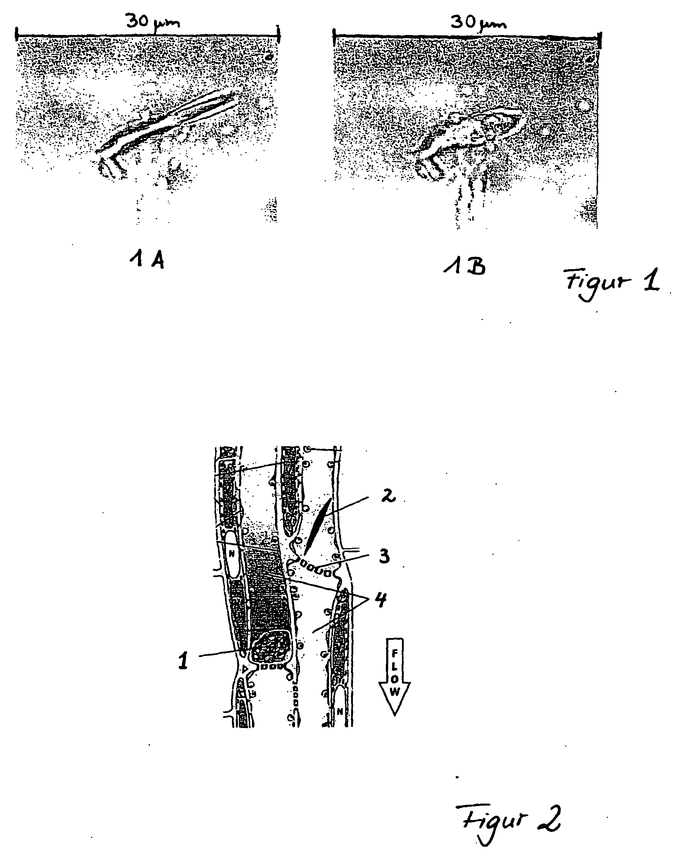

Das Phloem der höheren Pflanzen beinhaltet ein die Pflanze durchziehendes System von Mikrokanälen (Siebröhren), das dem Transport von Photoassimilaten dient (siehe Figur 2). Die Siebröhren bestehen aus Zellen, die im Verbund ein Kapillarsystem durch die gesamte Pflanze bilden, nämlich eben dieses Phloem. Dabei handelt es sich um ein Mikrofluidiksystem mit bis zu 3,5 MPa Innendruck und maximalen Strömungsgeschwindigkeiten bis zu 3 cm/min⁻¹.

Kristalline P-Proteine sind spezifische Inhaltskörper der Siebelemente der Leguminosen (Fabaceae). Sie wurden zunächst in Planzenschnitten beobachtet und als meist längliche, bis zu 30 µm große, kompakte Gebilde mit hochgeordneter ("kristalloider") Ultrastruktur beschrieben; ihre Funktion blieb jedoch für mehr als ein Jahrhundert nach ihrer Entdeckung unklar (Behnke, H.D., Nondispersive protein bodies in sieve elements: a survey and review of their origin, distribution and taxonomic significance. IAWA Bull.12, 143-175 (1991)). Inzwischen weiß man, daß sie schlagartig beim Auftreten drastischer Änderungen des hydrostatischen Drucks im Inneren der Siebröhren deformieren. Die Proteine gehen dabei vom geordneten "kristalloiden" Zustand (spindelförmig) (siehe Bezugszeichen 2 in Figur 2) in eine ultrastrukturell weniger geordnet wirkende, "disperse" Konformation über (siehe Bezugszeichen 1 in Figur 2; in dieser Figur bezeichnet 3 eine Siebplatte und 4 die Siebelemente). Die disperse Form besitzt eine abgerundete Gestalt und bildet Pfropfen in den Siebröhren und reduziert damit den weiteren Fluß der an Photoassimilaten reichen Flüssigkeit innerhalb des Röhrensystems. Die Proteinkörper verlieren hierbei ihre interne Organisation offenbar jedoch nicht vollständig, da sie in der Lage bleiben, den geordneten "kristalloiden" Zustand spontan wiederherzustellen. Eine Arbeitsgruppe um die Erfinder konnte zeigen, daß sich die *in situ* beobachteten Konformationsänderungen durch die Abhängigkeit der Konformation kristalliner P-Proteine von bestimmten divalenten Kationen hinreichend erklären lassen (Knoblauch, M., Peters, W.S., Ehlers, K. & van Bel, A.J.E.; Reversible calcium-regulated stopcocks in legume sieve tubes. Plant Cell 13, 1221-1230 (2001)). Der "kristalloide" Zustand herrscht bei Ca⁺⁺-Konzentrationen von deutlich unter 1 µM, wie sie mit Chelatoren eingestellt werden können, vor, während Konzentrationen im mikromolaren Bereich zur Transformation in die "disperse" Konformation führen. Im Hinblick auf die biologische Funktion der Proteinkörper wurde ihnen der Name "Forisomen" verliehen, abgeleitet von foris (lat.: Türflügel) und soma (griechisch: σωµα: Körper).

Aufgabe der vorliegenden Erfindung war es, die bisher nur *in situ* beobachteten "P-Proteine" zu isolieren, zu charakterisieren, ein reproduzierbares Verfahren für ihre Bereitstellung anzugeben und zu überprüfen, ob und ggf. für welche Verwendungszwecke sie sich eignen könnten.

Den Erfindern ist es in Lösung der gestellten Aufgabe nun erstmals gelungen, Forisomen aus Pflanzen der Familie der Leguminosen (Fabaceen) zu isolieren. Diese Isolierung hat sich als äußerst schwierig erwiesen. Zwar ist es möglich, einzelne Proteinkörper manuell aus der Zelle zu schieben und auf einen Objektträger zu übertragen; hierbei werden diese jedoch in unkontrollierbarer Weise zerstört, so daß die erhaltenen, mehr oder weniger amorphen Klumpen nur eine geringe Reaktivität zeigen, die sich in scheinbaren Volumenänderungen als Reaktion auf Änderungen der Calcium-Konzentrationen im Medium äußert. Forisomen aus solchen Pflanzen zu gewinnen, zu isolieren und in intakter Form zu beobachten, gelang jedoch erst nach vielen fehlgeschlagenen Versuchen und Vorversuchen.

Erfindungsgemäß wurde schließlich festgestellt, daß es günstig ist, das als Ausgangsmaterial für die Gewinnung der Forisomen einzusetzende Phloem mechanisch dadurch zu gewinnen, daß Längsabschnitte aus Stengeln oder dergleichen der Donorpflanze von Xylem befreit werden und anschließend das Phloem vom Cortex und vom Sklerenchym getrennt wird. Die Zellen des Phloems werden aufgeschlossen, und eine Suspension der aufgeschlossenen Zellen wird filtriert, wobei die Poren des Filtermediums vorzugsweise eine Größe besitzen, die intakten Zellen den Durchtritt verwehrt. Nach Gewinnung der die Forisomen enthaltenden Fraktion aus dem Filtrat können diese mit Hilfe einer Gradientenzentrifugation von den übrigen Bestandteilen getrennt werden.

Zur Aufbewahrung der gewonnenen Forisomen eignet sich ein Medium wie beispielsweise 70%-ige Saccharose bzw. 70% Glycerin in V-Medium (Zusammensetzung siehe Beispiel 1) (w/v); die Lagerfähigkeit ist bei -20C° sehr hoch; bei Raumtemperatur beträgt sie etwa 3 Wochen.

Die so isolierten, ggf. gelagerten Forisomen sind vollständig intakt. Sie weisen als Ganzes eine ausgeprägte Reaktivität auf. Dabei hat sich gezeigt, daß der "disperse" Zustand *in vitro* ebenso gut sicht- und manipulierbar ist wie der "kristalloide" Zustand. Die Änderungen der optischen Eigenschaften des Proteinkörpers beim Übergang zwischen "kristalloider" und "disperser" Konformation stellt einen unabhängigen Beleg für die dabei auftretenden Änderungen der inneren Organisation des Proteinkörpers dar.

Wie nun an unbeschädigt isolierten Proteinkörpern erkennbar wurde, geht mit der "Dispersion" nicht nur eine Verdickung des Proteinkörpers in den Richtungen senkrecht zu seiner Längsachse einher, sondern auch eine Kontraktion entlang der Längsachse (siehe Figur 1). Die Induktion dieser Konformationsänderung kann durch eine Gabe von Calciumionen induziert werden, mit der die Umgebungskonzentration an diesen Ionen von unter etwa 30nM Ca²⁺ auf vorzugsweise deutlich über diesen Wert angehoben wird (Schwellenkonzentration). Die Reaktion tritt ohne meßbare Verzögerung zeitgleich mit der Änderung der Calcium-Konzentration auf; sie ist vollständig, eine weitere Erhöhung der Calcium-Konzentration bewirkt keine Veränderung mehr. Die Verdickung ist uneingeschränkt reversibel und praktisch beliebig oft wiederholbar (experimentell wurden mindestens 50 Zyklen überprüft). Die Umkehr der Konformationsänderung wird durch eine Senkung der Calciumionen-Konzentration in der Umgebung der Forisomen auf unter 30nM bewirkt, z.B. durch Medienwechsel oder Zugabe von Chelatbildnern für das Calcium. Die Kontraktion infolge der Konformationsänderung liegt bei bis zu ca. 30%; dies ist ein bemerkenswert hoher Wert im Vergleich zu anderen aktuatorischen Proteinen. Die Konformationsänderung erfolgt im Millisekundenbereich.

Die unterschiedliche Reaktion völlig intakter "Kristalloide" im Vergleich zu den zuvor isolierten, beschädigten, amorphen Klumpen deutet an, daß die Fähigkeit zur Kontraktion eine weitgehend intakte interne Organisation voraussetzt. Dies bedeutet jedoch nicht, daß nur originäre "Kristalloide" zur Reaktion befähigt sind. Vielmehr konnten die Erfinder zeigen, daß auch Bruchstücke, die durch Zerschmettem gefrorener Proteinkörper gewonnen wurden, nach dem Auftauen in "gewohnter Weise" reagieren. Partien der "Kristalloide" reagieren also autonom, sofern nur die entsprechende innere Strukturierung gegeben ist. Erfindungsgemäß konnte festgestellt werden, daß diese Reaktivität ausgehend von intakten, in der Regel etwa 30-40µm langen und etwa 5-10µm dicken Forisomen mindestens bis zu einer Zerkleinerung in den Bereich, der lichtmikroskopisch gerade noch nachzuweisen ist, erhalten bleibt. Das heißt, daß selbst Forisomen-Bruchstücke mit Abmessungen im Bereich von etwa 1µm das beschriebene Verhalten aufweisen.

Calcium kann, ähnlich wie *in situ,* durch Barium und Strontium funktionell ersetzt werden; Magnesium bleibt auch hier ohne jede Wirkung.

Überraschend konnte nun festgestellt werden, daß kristalline P-Proteine *in vitro* auch auf Änderungen des pH-Wertes reagieren. Steigt der pH-Wert auf über etwa 9,5, insbesondere über pH 9,9, so stellt sich eine reversible Schwellung der Proteinkörper ein. Die Reaktion nimmt stufenweise in der Intensität bis zu einem pH-Wert von 10,6 zu. Fällt der pH-Wert unter einen Wert von 9,5, verschwindet die Schwellung; es gibt keine Hysterese. Ab pH-Wert 10,9 denaturieren die Forisomen. Die durch basische pH-Werte induzierte Schwellung geht, anders als bei der Calciumionengabe, mit einer Kontraktion in Richtung der Längsachse von nur wenigen Prozent einher, so daß im Endeffekt eine Volumenvergrößerung beobachtet wird. Sie unterscheidet sich daher von den durch Kationen bewirkten Konformationsänderungen. Im sauren Bereich werden die P-Proteine bei einem pH-Wert von etwa 4,5 irreversibel denaturiert.

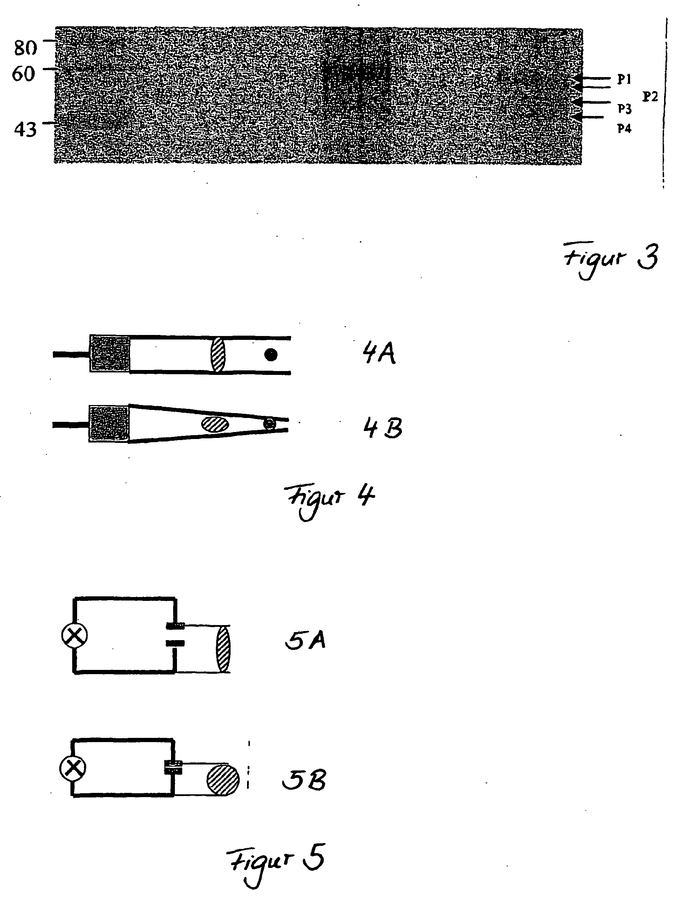

Die Forisomen der vorliegenden Erfindung weisen zwei unterschiedliche Proteine auf. Dies konnte anhand einer Polyacrylamid-Gelelektrophorese des Proteinmaterials festgestellt werden, das wie oben beschrieben mit Hilfe einer Gradientenzentrifugation isoliert worden war (siehe Figur 3). Die Molekulargewichte der beiden dabei aufgefundenen Proteine liegen im Bereich von ca. 55 bis 65 kDa (P1) und 53 bis 63 kDa (P2). Die genannte Polyacrylamid-Gelelektrophorese liefert weitere Proteinbanden, die weiteren Proteinen (P3 und P4) zugeordnet werden können, bei denen es sich aber um Abbauprodukte von P1 und P2 handelt. Diese Hypothese wird durch Antikörperstudien gestützt. Eine enzymatische Verdauung der Proteine P1 und P2 liefert zwei Peptide, die über Tandem-Massenspektrometrie (CID-MS/MS) analysiert werden konnten. Diese beiden Peptide haben die folgenden Strukturen:

Bei weiteren Versuchen konnte außerdem festgestellt werden, dass das Protein P1 die folgenden Aminosäuresequenzen umfasst:

Erfindungsgemäß wurden also Forisomen oder Proteinkörper aus den Siebröhren der Leguminosen aufgefunden, die mindestens aus zwei verschiedenen Proteinen vergleichbarer Größe bestehen und bereits in sehr gering dimensionierten Aggregaten oder Komplexen reversible kontraktile Eigenschaften aufweisen, welche sich zum einen durch Calciumionen oder andere, vergleichbare zweiwertige Ionen induzieren lassen, zum anderen auch durch eine Veränderung des pH-Wertes; wobei bemerkenswert ist, daß der pH-Wert-Bereich, in dem die Konformationsänderung auftritt, unphysiologisch ist und daß die beiden beobachteten Konformationsänderungen unterschiedlich sind. Für die Auslösung der Konformationsänderung ist kein ATP als Energielieferant notwendig. Bezüglich der Kontraktiliät in Gegenwart von Calciumionen ähneln die Proteinkörper in gewisser Weise denen des Spasmins sessiler Ciliaten, dessen Kontraktion ebenfalls calciumabhängig ist. Allerdings kontrahiert Spasmin isotrop und reagiert erst bei hundertfach höheren Calcium-Konzentrationen.

Aufgrund der Reaktivität sehr kleiner Proteinkörper mit einem Durchmesser von nur etwa einem Mikrometer oder sogar weniger kann spekuliert werden, daß während der Ontogenese der Siebelemente anfänglich sehr kleine Proteinkörper mit einem entsprechend kleinen Durchmesser durch Anlagerung weiterer Untereinheiten zu dem Komplex im Mikrometerbereich kondensierten, der sich heute auffinden läßt. Diese würde erklären, warum der Proteinkörper trotz variabler Größe in allen Stadien reaktiv ist.

Bioanaloge Aktuatoren sind in der Bionik im mikro- und nano-Bereich von zunehmendem Interesse. Mit ihrer mechanischen Aktivität, spezifischen Reaktivität, Langlebigkeit und offensichtlichen Ungefährlichkeit für den menschlichen Organismus (Leguminosen sind wichtige Nahrungsmittel) verfügen die Proteinkörper der vorliegenden Erfindung über Eigenschaften, die für einen Mikro- bzw. Nanoaktuator besonders in der Medizintechnik gefordert werden. Gegenüber anderen kontraktilen Elementen des Cytoskeletts besitzen sie den Vorteil, daß ihre strukturelle Kohärenz nicht auf kontinuierlichem Auf- und Abbau beruht. Andere Motorproteine des Cytoskeletts unterliegen nämlich einem ständigen Turnover, was in der Regel ihre Haltbarkeit *in vitro* stark begrenzt.

Die Erfinder konnten zeigen, daß sich die Kontraktion der erfindungsgemäßen Forisomen quantifizierbar in Krafterzeugung umsetzen läßt. Die Tatsache, daß auf Glas- oder anderen Oberflächen gepresste Forisomen an diesen anhaften, ermöglicht z.B. die Fixierung individueller Exemplare zwischen zwei Federarmen, z.B. aus Glasfasern. Die Hafteigenschaften der Forisomen an den Federarmen können durch eine geeignete Beschichtung des Materials der Federarme, z.B. mit poly-L-Lysin oder Klebstoffen wie z.B. Kwik-Sil (Handelsmarke der World Precision Instruments, Sarasota, FL, USA), ggf. verbessert werden. Wird durch eine Gabe von Calciumionen die Kontraktion ausgelöst, so entwickelt jeder einzelne Proteinkörper eine so große mechanische Kraft, daß die Federarme erheblich verbogen werden. Eine solche Anordnung ist daher als Mikropinzette geeignet, mit der sich organische oder anorganische Strukturen wie Zellen, Gewebe oder Molekülkörper manipulieren oder positionieren lassen. In Figur 4 ist eine solche Pinzette schematisch dargestellt: 4A bezeichnet den offenen Zustand mit relaxiertem Forisom, 4B bezeichnet den geschlossenen Zustand mit kontrahiertem Forisom. Die Bewegung der Pinzette kann manuell unter dem Lichtmikroskop oder elektronisch gesteuert werden. Da die Größe funktionsfähiger Forisomen im Bereich von 1µm (oder sogar darunter) bis 40µm liegt, kann die Spannweite der Pinzettenspitze in einem entsprechenden Bereich eingestellt werden. Mit einer solchen Pinzette können kleinste Körper oder Substanzmengen zielgenau transportiert und zur Reaktion und biologische Prozesse ortsgenau zum Ablauf gebracht werden. Alternativ lassen sich Forisomen beispielsweise als Sensorelemente für einen pH-Wert-Umschlag im Bereich bei etwa 9,5 bis 10 und für die Erhöhung der Calciumionen in einem Medium von unter auf über 30 nM oder umgekehrt verwenden, die gleichzeitig als Anzeigenelemente nutzbar sind: Forisomen können so zwischen zwei Federarmen angeordnet werden, dass ihre Kontraktion unter den genannten Umgebungsänderungen einen Kontakt der Spitzen dieser Federarme bewirkt. Wenn die Spitzen elektrisch leitend sind und über die Federarme beim Kontakt der Spitzen ein Stromkreis geschlossen wird, kann die Änderung der Wasserstoff- bzw. Calciumionen-Konzentration im Medium, z.B. in einem enzymatischen Verfahren, unmittelbar detektiert werden. Ein derartiges Element ist schematisch in Figur 5 gezeigt: 5A bezeichnet den offenen Schaltkreis mit relaxiertem Forisom, 5B den geschlossenen Schaltkreis mit kontrahiertem Forisom.

Nachstehend soll die Erfindung anhand von Beispielen und Vor- und Vergleichsbeispielen näher erläutert werden.

### Beispiel 1

Aufreinigung von Forisomen aus Vicia faba:

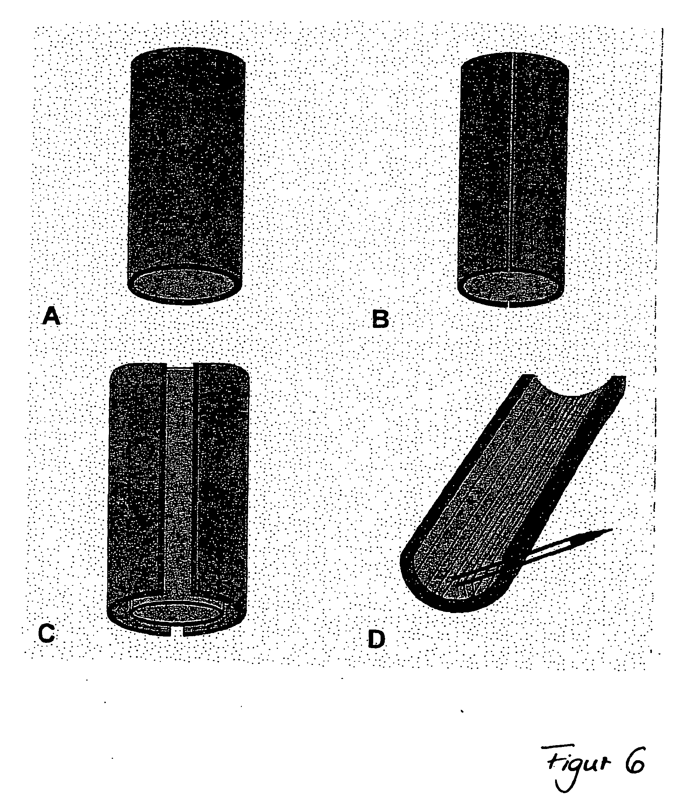

Vorbemerkung: Vicia faba L. cv Witkiem major (Nunhem Zadenh BV, Haelen, The Netherlands) oder andere Vicia faba-Sorten lassen sich problemlos im Gewächshaus züchten.
1. Abtrennen der Wurzel vom Sproß am Hypokotyl: Die Sproßachsen 7-8 Wochen alter Vicia faba-Pflanzen wurden kurz oberhalb der Bodenlinie mit einer Rasierklinge abgetrennt, anschließend alle Blätter entfernt und 10 cm der Sproßspitze abgeschnitten.
2. Trennen des Cortex inklusive Phloem vom Xylemcylinder: Methode zur Trennung des Phloem vom Rest der Sproßachse (die intakte Sproßache ist in Figur 6A gezeigt). Als effektivste Technik erwiesen sich zwei gegenüberliegende Schnitte entlang der Längsachse der Pflanze, die die Rinde bis zum Xylem der Leitgewebe, die hier ringförmig angeordnet sind, durchtrennten (sh. Figur 6B). Anschließend wurde die Rinde entlang des Xylems vorsichtig abgezogen (Figur 6C). Dabei konnte beobachtet werden, daß das Phloem auf der Innenseite der abgezogenen Rinde haften blieb und sich somit sauber vom Xylem trennen ließ. Besonderes Augenmerk mußte auf die Bereiche um die Nodien gerichtet werden, da hier die Leitgewebe abzweigen und somit an diesen Stellen eine saubere Trennung schwer zu erreichen ist. Anschließend konnte der innere Xylemzylinder verworfen werden. Vicia-Pflanzen, bei denen das sekundäre Dickenwachstum noch nicht eingesetzt hatte, zeigten eine ringförmige Verteilung von Sklerenchymfasem im Cortex, denen das Phloem aufgelagert war. Aufgrund der Festigkeit dieser Fasern konnten sie leicht mit Hilfe einer Pinzette vom Cortex abgezogen werden (Figur 6D), wobei das Phloem an ihnen haften blieb.
3. Einlegen des Cortex in V-Medium (10 mM EDTA, 10 mM Tris, 100 mM KCI pH 7.3): Eine vorangestellte einstündige Inkubation in V-Medium erleichtert die Trennung von Phloem und Sklerenchym (Schritt 4) und ermöglicht zudem den Forisomen eine Regeneration in die kristalline Form.
4. Trennen des Phloems vom Sklerenchym und Cortex: Das Phloem wird nach Ablauf der Inkubationszeit mit Hilfe eines stumpfen Skalpells von der Innenfläche der Rinde abgetragen.
5. Mörsern des Phloems unter flüssigem Stickstoff (vorteilhaft ist zwischenzeitliches Sieben, um bereits freigesetzte Forisomen vor Zerstörung zu schützen): Das Phloem wurde mit Zellstoff trockengetupft und unter flüssigem Stickstoff etwa 10 Minuten bis zum Erreichen eines pulvrigen Zustandes gemörsert und in V-Medium aufgenommen. Die Suspension wurde danach durch ein Gewebesieb gegossen und der zurückbleibende Überstand mehrmals mit 5 ml V-Medium gründlich durchgespült. Die Porengrößen des Gewebesiebs wurden variiert und dabei die Größen 100 µm, 80 µm und 59 µm getestet. Die Porengröße von 59 µm erwies sich als optimal, da die Forisomen mit einer Länge von ungefähr 30 µm problemlos durchfallen konnten, auch wenn sie noch mit anderem kleinkörnigem Material zusammenhafteten. Darüber hinaus verwehrte diese Porengröße unaufgeschlossenen Zellen die Passage.
6. Das Filtrat aus Schritt 5. mit den darin enthaltenen Forisomen wurde bei 5000 x g und 4°C 10 Minuten lang zentrifugiert. Der Überstand wurde verworfen.
7. Das bei der Zentrifugation entstandene Pellet wurde in 5 ml V-Medium rückgelöst und auf einen Nycodenz Gradienten (80 - 20 % in V-Medium) aufgetragen: Zur Vorbereitung der Gradientenzentrifugation wurde mittels eines Gradientengießers ein kontinuierlicher Gradient gegossen. Zu diesem Zweck wurde eine Säule mit 15 ml 80%iger Nycodenzlösung befüllt, während die andere Säule mit 3,75 ml 80%iger Nycodenzlösung und mit 11,25 ml Pflanzensuspension befüllt wurde - entsprechend einer 20%igen Nycodenzlösung. Mit Hilfe des Gradientengießers konnte nun ein Gradient von 80% am Boden bis 20% am oberen Rand des Zentrifugenröhrchens erreicht werden.
8. Der Nycodenzgradient gemäß Schritt 7 wurde 3h bei 150000 x g in der Ultrazentrifuge zentrifugiert. Mit Hilfe dieser Zentrifugation erhielt man ein Bandenmuster wie in Figur 7A zu sehen: Mit der Bezugsziffer 1 ist eine Bande aus Chloroplasten als oberste Schicht zu sehen. Im mittleren Bereich befindet sich beim Bezugszeichen 2 eine breite, relativ scharf abgesetzte Bande, die hauptsächlich aus Membranresten besteht. Am Boden des Zentrifugenröhrchens, mit 4 bezeichnet, befindet sich ein Pellet aus Zellwandtrümmern. Etwa im oberen Drittel der Phase zwischen Membranbande und Zellwand-Pellet kann eine dünne Bande identifiziert werden, die ausschließlich aus Forisomen besteht (in Figur 7A mit Bezugsziffer 3 bezeichnet).
9. Die abgenommene Fraktion (forisomenhaltig) wurde mit einem doppelten Flüssigkeitsvolumen versetzt und bei 5000 x g 10 min. zentrifugiert. Der Überstand wurde verworfen.
10. Zur Aufbewahrung werden die Forisomen mit 70% Saccharose in V-Medium (w/v) bei minus 20°C eingefroren.

Nycodenz besitzt die folgende Formel:

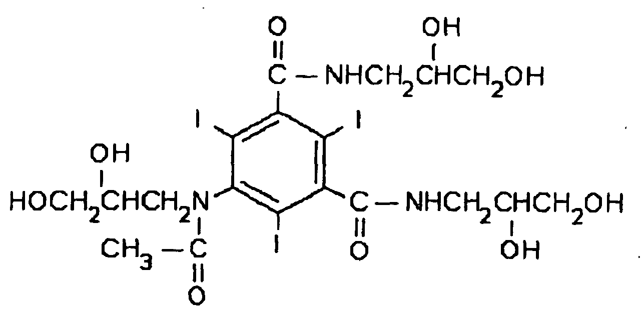

Die Verbindung findet Verwendung als nichtionisches Dichtegradientenmedium. Literatur: Rickwood, D., et al., Anal. Biochem. 123, 23 (1982) Beilstein Registry Number: 2406632
Vertrieb: Nycomed Pharma AS, Norwegen.
Synonym Histodenz (Sigma-Aldrich)

### Beispiel 1 A:

Beispiel 1 wurde mit gleichaltrigen Vicia-Pflanzen wiederholt, deren sekundäres Dickenwachstum bereits eingesetzt hatte. Die Trennung des Cortex und Phloem vom Xylemzylinder erfolgt hier folgendermaßen:

Bei diesen Pflanzen schloß sich das Phloem durch die Bildung eines interfascikulären Cambiums zu einem Zylinder. Die Sklerenchymfasem waren ebenfalls stärker ausgeprägt, so daß nach Trennung des Cortex vom Xylem ein Abziehen der Sklerenchymfasem mit Hilfe einer Pinzette nur schwer durchzuführen und mit einem hohen Verlust an Phloem verbunden war. Bei diesen Pflanzen erwies es sich als wesentlich effektiver, das Phloem nach Ablauf der Inkubationszeit mit Hilfe eines stumpfen Skalpells von der Innenfläche der Rinde abzutragen. Dies hat den Vorteil, auch die Sklerenchymfasem abtrennen zu können. Außerdem ist die Ausbeute an Phloem deutlich höher als bei den Vicia-Pflanzen ohne sekundäres Dickenwachstum.

### Beispiel 1B:

Beispiel 1 wurde wiederholt, wobei die Gradientenzentrifugation gemäß den Schritten 7 und 8 mit einer Pflanzensuspension erfolgte, der vor dem Gießen des Gradienten 0,1 % Triton X100 zugegeben worden war, um Membran- und Zellwandproteine herauszulösen und möglicherweise entstehende Wechselwirkungen zwischen Forisomen mit Membranresten herabzusetzen. Nach Zentrifugation unter denselben Bedingungen erhielt man dabei eine Bandenverteilung gemäß Figur 7B: Zwar konnte hier keine Chloroplastenbande mehr beobachtet werden, allerdings befanden sich die Forisomen trotz der Behandlung mit Triton zu einem überwiegenden Teil in der Membranbande (1).

### Beispiel 2:

Auftrennung der Forisomen mittels SDS-Polyacrylamid-Gelelektrophorese
1. Die Forisomen wurden in V-Medium für 10 min. bei 5000 x g zentrifugiert.
2. Der Überstand wurde verworfen, und die Forisomen wurden in 250µl 50mM Tris-HCl Puffer pH 6,8 aufgenommen.
3. Die Forisomenlösung wurde mit 200 µl 10 % Natrium-dodecylsulfat-Lösung, 25 µl 60 % Saccharose-Lösung und 2,5 µl 2-Mercaptoethanol versetzt und 5 min. bei 95 °C denaturiert.
4. Die Mischung wurde 5 min. bei 12000 x g zentrifugiert.
5. Die denaturierten Proteine wurden mit Hilfe eines 10 % SDS-Polyacrylamidgels nach Laemmli (1970) elektrophoretisch aufgetrennt.
6. Die Proteine wurden im Anschluss durch Coomassiefärbung sichtbar gemacht. Figur 3 zeigt das Gel mit den aufgetrennten Forisomenbestandteilen. Das Molekulargewicht ist in KDa angegeben.

### Beispiel 3:

Bestimmung der Peptidsequenzen mittels Massenspektrometrie
1. Enzymatischer Verdau mit Trypsin
   Die Proteinbanden der SDS-PAGE gemäß Beispiel 2 werden aus dem Gel ausgeschnitten und in ein Eppendorfgefäß überführt. Nachdem die Gelstückchen mit Acetonitril dehydriert wurden, wird das Acetonitril entfernt. Anschließend werden die Gelstückchen in einer Vakuumzentrifuge getrocknet. Darauf wird 10 mM Dithiotreitol (DTT) in 100 mM NH₄HCO₃ dazugegeben, bis alle Gelstückchen mit der Lösung vollständig bedeckt sind. Die Proteine werden nun 1 h bei 56 °C reduziert. Nach Kühlung auf Raumtemperatur wird die DTT Lösung durch dasselbe Volumen von of 55 mM lodoacetamide in 100 mM NH₄HCO₃ ersetzt. Nach 45 min. Inkubation bei Raumtemperatur und Dunkelheit werden die Gelstückchen mit 50-100 µL 100 mM NH₄HCO₃ 10 min lang gewaschen, mit Acetonitril getrocknet, mit 100 mM NH₄HCO₃ rehydriert und wiederum mit demselben Volumen an Acetonitril getrocknet. Die flüssige Phase wird entfernt und die Gelstückchen werden in einer Vakuumzentrifuge vollständig getrocknet. Daraufhin werden die Gelstückchen mit dem "Verdaupuffer" (50 mM NH₄HCO₃, 5 mM CaCl₂ und 12.5 ng/µL) Trypsin) bei 0°C versetzt. Nach 45 min. werden die Überstände entfernt und durch 5-10 µL eines Puffers, bestehend aus 50 mM NH₄HCO₃ und 5 mM CaCl₂ ersetzt, um die Gelstückchen während des Verdaus für 12 h bei 37°C feucht zu halten. Schließlich werden die entstandenen Peptide durch Extraktion mit 20 mM NH₄HCO₃ und dreimaliger Extraktion mit einer Mischung aus 5% Ameisensäure 45 entsaltztem Wasser und 50% Acetonitrile (je 20 min) bei Raumtemperatur und die gesammelten Extrakte in einer Vakuumzentrifuge vollständig getrocknet.
2. Entsalzung der Proben
   Die Probe wird in 10 µl 1 % TFA aufgenommen und anschliessend mit ZipTipsTM (ZipTipsTM ist ein eingetragenes Warenzeichen der Millipore Corporation, Bedford, MA oder einer Tochtergesellschaft) entsalzt.
   Zuerst erfolgt die Äquilibrierung der Spitze mit 10µL einer Mischung von 50% Wasser (HPLC grade) und 50% Acetonitril (HPLC grade) und anschließender Abgabe. Dieser Vorgang wird einmal wiederholt. Die Spitze wird darauf mit 10 µl 0, 1 % iger Trifluoressigsäurelösung befeuchtet, indem man zweimal diese Lösung einsaugt und wieder abgibt.
   Die Peptidmischung aus dem Verdau mit Trypsin wird nun vollständig eingesaugt und wieder abgegeben. Dieser Vorgang wird 9mal wiederholt.
   Die gebundenen Peptide werden durch 10maliges Waschen mit 0,1% Trifluoressigsäurelösung vom Salz befreit. Schließlich werden die Peptide mit 3-10 µl einer Mischung aus 0,1 %iger Ameisensäure, 39,9 % Wasser (HPLC grade) und 60% Acetonitril (HPLC grade) eluiert. Das Eluat wird je 4 mal wiederum eingesaugt und abgegeben.
   3 µl des Eluats werden in eine Nanospray-Glaskapillare gefüllt. Die Glaskapillare wird in den dafür vorgesehenen Halter befestigt und der Halter in die Nanospraylonenquelle überführt.
   Die Kalibrierung des Massenspektrometers erfolgt für ein Massenspektrum mit einer Peptidmischung (je 1 µmol/l Angiotensin l, Substance P, GluFibrinopeptide, Renin Substrate, ACTH Clip 18-39 und Rinder-Insulin) und für den MS/MS-Modus mit dem Peptid GluFibrinopeptide (100 nmol/l).
3. Aufnahme der Massenspektren bzw. CID-MS/MS Spektren
   Alle Massenspektren und MS/MS werden mit einem Q-TOF-2™ der Firma Micromass aufgenommen. Für die Ansteuerung des Massenspektrometers und der Datenprozessierung wird die Software Masslynx 3.5™ und ProteinLynx 1.0™ verwendet.
   Die Kapillarspannung beträgt zwischen 1,0-1,5 kV, die Cone Spannung wird im Massenbereich m/z 400-2500 von 20 eV-100 eV variiert.
   Der Argon-Druck in der Kollisionszelle beträgt bei CID-MS/MS (5+1) 10-6 mbar. Die Aufnahmezeit beträgt pro Scan 2,4 sec. mit einem Zeitraum von 0,1 sec. zwischen jedem scan.
   Für die De Novo Sequenzierung werden alle gebildeten Peptid-Ionen von dem enzymatisch verdauten Proteins einzeln und vollautomatisiert mit Hilfe der "Collision-Induced Dissociation" (CID) selektiert und fragmentiert (CID-MS/MS). Dabei werden auschließlich 2, 3 und 4 fach protonierte Spezies selektiert. Aus dem gewonnenen MS/MS-Spektrensatz werden Sequenz-Informationen der entsprechenden Peptide softwaregestützt ermittelt. Diese Sequenz-informationen werden für eine "Blast"-Suche in der Mascot Datenbank verglichen, um homologe Proteine zu finden. Alle MS/MS Spektren und die daraus resultierenden Aminosäuresequenzen der entsprechenden Peptide werden evaluiert.

### Beispiel 4:

Induktion der Kontraktion von Forisomen durch Calciumionengabe.

Isolierte Forisomen werden in eine 1 mL fassende Versuchskammer übertragen. Dabei wird jeweils ein isoliertes Forisom mit Hilfe einer Mikroinjektionsnadel an einem Ende an den Kammerboden gepresst, um ein Verdriften beim Durchspülen der Kammer zu verhindern. Anschließend werden mit Hilfe einer automatischen Durchflußvorrichtung wechselweise V-Medium (10 mM EDTA, 10 mM TRIS pH 7,3, 100 mM KCI)und Ca-Medium (10 mM CaCl₂, 10 mM Tris-HCl pH 7,3,100 mM KCI) auf das Forisom aufgebracht, was jeweils zu einer Konformationsänderung führt. Die Konformationsänderung kann mikroskopisch mittels einer Kamera beobachtet werden, die auf dem Beobachtungsmikroskop aufgebracht ist. In Figur 1 sind die beiden Konformationen gezeigt, die das Forisom unter V-Medium, also im relaxierten Zustand (Figur 1A) und unter Ca-Medium, also im kontrahierten Zustand (Figur 1 B) annimmt. Das Mikroskop befand sich bei den Aufnahmen im Durchlicht-Interferenzkontrast-Modus. Die Breite der Abteilungen entspricht jeweils 30 µm.

### Beispiel 5:

Induktion der Volumenvergrößerung von Forisomen durch Erhöhung des pH-Wertes.

Ein isoliertes Forisom wird in eine 1 ml fassende Probenkammer eingebracht, in der sich V-Medium befindet. Dabei wird jeweils ein isoliertes Forisom mit Hilfe einer Mikroinjektionsnadel an einem Ende an den Kammerboden gepresst, um ein Verdriften beim Durchspülen der Kammer zu verhindern. Anschließend wird, ausgehend von pH 7,3, der pH-Wert mit Hilfe einer automatischen Durchflußvorrichtung in Stufen von 0,3 Einheiten erhöht, wobei das verwendete Medium calciumfrei bleibt. Eine erste Reaktion des Forisoms kann bei einem pH-Wert von 9,4 festgestellt werden. Die Intensität der Reaktion steigert sich mit zunehmendem pH-Wert bis zu pH 10,6. Die Reaktion ist jeweils nach Gaben von V-Medium pH 7 vollständig reversibel. Ab und über einem pH-Wert von 10,9 denaturiert das Forisom.

Werden anstelle von EDTA/TRIS andere Puffermedien verwendet, die gegenüber Calciumionen chelatisierende Eigenschaften besitzen, wird derselbe Reaktionsverlauf beobachtet.

Im sauren Bereich verändert sich die Struktur des Forisom beim Erreichen eines pH-Wertes von 4,9. Allerdings kann man keine schrittweise erfolgende Steigerung der Reaktion beobachten. Bereits bei einem pH-Wert von 4,6 ist das Forisom irreversibel denaturiert.

Die Konformationsänderung können mikroskopisch mittels einer Kamera beobachtet werden, die auf dem Beobachtungsmikroskop aufgebracht ist.

### Beispiel 6:

Herstellung einer Mikro- oder Nanopinzette zur Manipulation individueller Zellen oder Moleküle

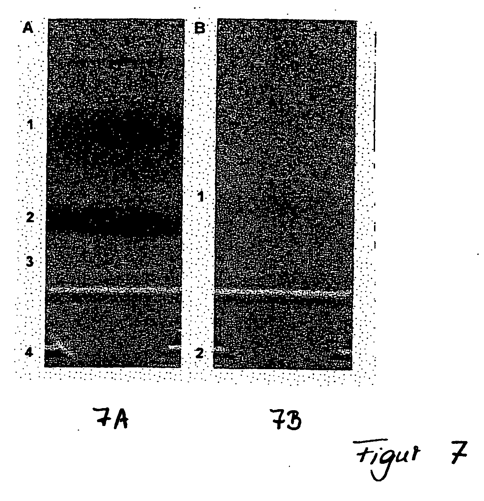

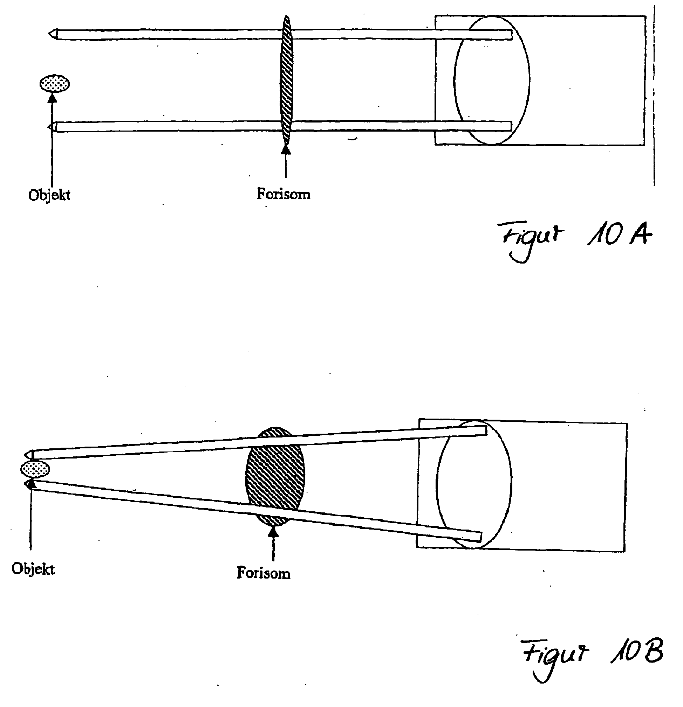

In Figur 8 ist eine Pinzettenanordnung gezeigt, in der zwei Federarme (z.B. Glasfasern oder -mikropipetten) in einem der Länge des Forisoms entsprechenden Abstand auf einem Träger positioniert und befestigt sind (z.B. durch einen Kleber). Dabei kann das Forisom beispielsweise an den Enden mit den Spitzen der Federarme durchstossen werden (siehe Figur 9A). Im Falle von Glasfederarmen werden dabei die natürlich beim Produktionsprozess entstehenden Spitzen genutzt. Bei anderen Materialien können Spitzen z.B. durch Ätzung hergestellt werden. Die Pinzette wird dann so in die Nähe des zu greifenden Objektes gebracht, dass dieses in der Nähe des Forisoms zwischen den Federarmen zu liegen kommt (siehe Figur 9A). Alternativ kann das Forisom durch Adhäsion an einer zentralen Stelle an den Federarmen befestigt werden, wobei die Federarme adhäsionssteigemd beschichtet sein können, sofern die Adhäsion des Federarms am Forisom nicht ausreichend ist. So können Glasoberflächen beispielsweise mit Poly-L-Lysin oder vergleichbaren Materialien beschichtet sein. Durch die zentrale Positionierung des Forisoms wird bei dieser Methode ein höherer Federweg der Arme erreicht (siehe Figur 10A). Je nach Material der Federarme ist wegen unterschiedlicher Federkonstanten deren Durchmesser, deren Länge oder die Position und/oder Größe des Forisoms anzupassen. In diesem Beispiel wurden die Federarme aus Borosilikatglas mit einem Durchmesser von ca. 1-2µm und einer Länge von ca. 100-500µm verwendet.

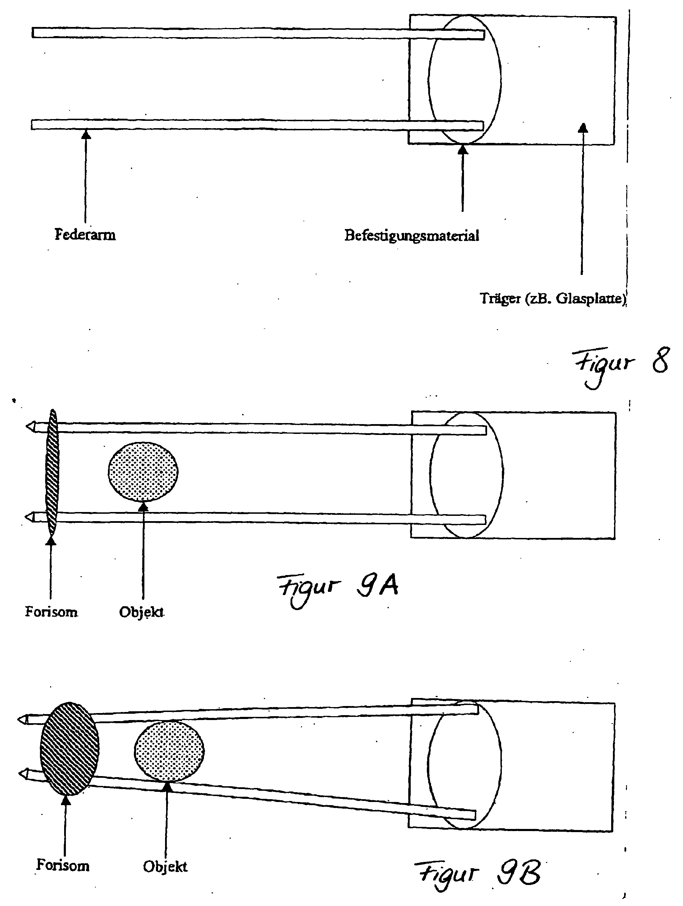

Die Ansteuerung des Forisoms kann je nach Anwendung auf verschiedene Art erfolgen. Durch Zugabe von freiem Ca⁺⁺ zur Lösung oder durch pH-Wert-Änderung wird eine Kontraktion bzw. eine Volumenvergrößerung ausgelöst. Als Medien hierfür eignen sich beispielsweise die unter den Beispielen 4 und 5 genannten. Die Kontraktion durch Ca⁺⁺-Zugabe bewirkt ein Schließen der Pinzette, wie in den Figuren 9B und 10B gezeigt.

### Beispiel 7:

Mikro- oder Nanoschalter zur Detektion von pH-Wert- und Calciumionen-Konzentrationsänderungen in einem Medium.

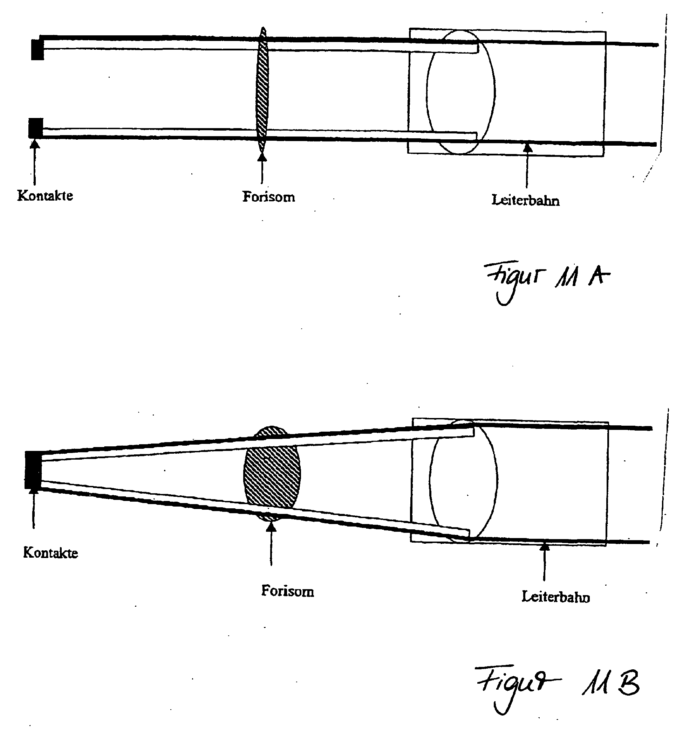

In ähnlicher Weise wie in Beispiel 6 für Pinzetten gezeigt können Mikro- oder Nanoschalter gebaut werden, die sich als Sensoren für die Anzeige einer pH-Wert-Änderung oder einer Änderung der Calciumionen-Konzentration in dem Bereich eignen, in dem die reversible Konformationsänderung der Forisomen erfolgt. Hierfür werden die Federarme aus entsprechenden Metallen, mit einem leitfähigen Material beschichtetem Glas oder dergleichen hergestellt, oder es werden Elektroden an die Spitzen angebracht (siehe Figur 11A). Durch die Konformationsänderung des Forisoms bei einer entsprechenden Veränderung des pH-Wertes bzw. der Calciumionen-Konzentration im das Forisom umgebenden Medium werden die Kontakte zusammengeführt (bzw. geöffnet), und es kommt zu einer deutlichen Abnahme (bzw. Zunahme) des Widerstandes im Stromkreis, der seinerseits mit herkömmlichen Mitteln als Schaltvorgang detektiert und ausgekoppelt werden kann (siehe Figur 11 B). Als Kontakte kommen idealerweise Silberelektroden oder Kupferelektroden (je nach Lösung) zum Einsatz. Für spezielle Einsatzfelder sind jedoch auch Kontakte aus anderen Materialien möglich.

## Patentansprüche

1. Proteinkörper (Forisom), **gekennzeichnet durch**:
- seine Herstellbarkeit aus Fabaceen und
- eine reversible, anisotrope Kontraktibilität mit der Fähigkeit, bei einer Erhöhung der umgebenden Calciumionen-Konzentration über einen Schwellenwert von ca. 30nM in den Richtungen senkrecht zur Längsachse dicker und entlang der Längsachse kürzer zu werden und umgekehrt, und der Fähigkeit, bei einer Erhöhung des umgebenden pH-Wertes auf über etwa 9,5 senkrecht zur Längsachse dicker zu werden, ohne daß er sich entlang der Längsachse entsprechend verkürzt, und umgekehrt.

2. Proteinkörper (Forisom) nach Anspruch 1, **gekennzeichnet durch** eine Länge von etwa 1µm bis zu etwa 40µm und senkrecht hierzu mit einem Durchmesser von etwa 1µm bis zu etwa 10µm.

3. Proteinkörper (Forisom) nach Anspruch 1 oder 2, **gekennzeichnet dadurch, daß** er ein erstes Protein (P1) mit einem Molekulargewicht im Bereich von ca. 55-65 kDa und ein zweites Protein (P2) mit einem Molekulargewicht in Bereich von 53-63 kDa umfasst.

4. Proteinkörper (Forisom) nach Anspruch 3, **dadurch gekennzeichnet, dass** sich aus den Proteinen bei tryptischer Verdauung die folgenden Peptide nachweisen lassen: und dass das Protein P1 weiterhin die folgenden Bruchstücke enthält:.

5. Proteinkörper (Forisom) nach einem der voranstehenden Ansprüche, **gekennzeichnet durch** eine Kontraktibilität entlang der Längsachse von bis zu etwa 30% bei gleichzeitiger Expansion senkrecht dazu um circa 100 %.

6. Protein P1 mit einem Molekulargewicht im Bereich von ca. 55-65 kDa wie in Anspruch 3 definiert oder Bruchstück hiervon, enthaltend die oder bestehend aus der folgenden Sequenz (von N-terminal nach C-terminal gelesen): und/oder enthaltend die oder bestehend aus der folgenden Sequenz (von N-terminal nach C-terminal gelesen):

7. Protein P2 mit einem Molekulargewicht im Bereich von ca. 53-63 kDa wie in Anspruch 3 definiert oder Bruchstück hiervon, enthaltend die oder bestehend aus der folgenden Sequenz (von N-terminal nach C-terminal gelesen): und/oder enthaltend die oder bestehend aus der folgenden Sequenz (von N-terminal nach C-terminal gelesen):

8. Protein P1 mit einem Molekulargewicht im Bereich von ca. 55-65 kDa wie in Anspruch 3 definiert oder Bruchstück hiervon, enthaltend die oder bestehend aus den folgenden Sequenzen (von N-terminal nach C-terminal gelesen):

9. Protein P1 oder Bruchstück hiervon nach Anspruch 8, weiterhin enthaltend mindestens eine der folgenden Aminosäuresequenzen: und

10. Verfahren zum Isolieren von Proteinkörpern (Forisomen) nach einem der Ansprüche 1 bis 5, **gekennzeichnet durch** die folgenden Schritte;
(a) Gewinnung des Phloems einer Pflanze aus der Familie der Fabaceen;
(b) Aufschließen der Zellen des Phloems;
(c) Filtrieren einer Suspension der aufgeschlossenen Zellen; und
(d) Abtrennen der Forisomen von den übrigen Bestandteilen der Suspension mit Hilfe einer Gradientenzentrifugation.

11. Verfahren nach Anspruch 10, **dadurch gekennzeichnet, daß** die Gradientenzentrifugation mit einer Nycodenzlösung erfolgt, wobei das Medium der Suspension gemäß Schritt (c) KCI in einem geeigneten Puffer enthält.

12. Verfahren nach Anspruch 10 oder 11, **dadurch gekennzeichnet, daß** die Pflanze aus der Familie der Fabaceen Vicia faba ist.

13. Verwendung einer oder mehrerer Proteinkörper (Forisomen) nach einem der Ansprüche 1 bis 5 als kontraktive(s) Element(e) in einer Mikro-Pinzette oder in einem Detektionselement für pH-Wert-Änderungen oder Änderungen der Calciumionen-Konzentration.

14. Verfahren zum Betreiben einer Mikropinzette, **dadurch gekennzeichnet, dass** ein Forisom geeigneter Größe gemäß einem der Ansprüche 1 bis 5 stabil zwischen zwei Federarmen angeordnet wird und anschließend die Konzentration an Calciumionen in der Umgebung des Forisoms von deutlich unter 30nM auf deutlich über 30nM erhöht wird.

15. Verfahren zum Betreiben eines Anzeigeelementes für eine Veränderung der Calciumionen von deutlich unter 30 nm auf deutlich über 30 nm oder für eine Veränderung des pH-Wertes von unter pH 9,4 auf über pH 10,0 in einem Medium, **dadurch gekennzeichnet, dass** ein Forisom geeigneter Größe gemäß einem der Ansprüche 1 bis 6 stabil zwischen zwei Federarmen angeordnet wird, deren Spitzen sich bei einer Kontraktion des Forisoms berühren können, wobei die Federarme und ihre Spitzen so ausgestaltet sind, dass bei der genannten Berührung ein Stromkreis geschlossen wird und Strom fließt, wobei der Stromfluss oder die Unterbrechung des Stromkreises als Signal dafür detektiert wird, dass die Calciumionen-Konzentration auf deutlich über 30 nm steigt oder umgekehrt bzw. der pH-Wert auf über pH 10,0 steigt oder unter 9,4 fällt.

## Revendications

1. Corps protéique (forisome)
**caractérisé par**
- son aptitude à être préparé à partir de fabacées, et
- une contractilité anisotrope réversible ayant la capacité, dans le cas d'une augmentation de la concentration en ions calcium environnante, de grossir au-delà d'une valeur de seuil d'environ 30 nM dans les directions perpendiculaires à l'axe longitudinal et de se raccourcir le long de l'axe longitudinal et inversement, et la capacité, dans le cas d'une augmentation du pH environnant à plus de 9,5 environ, de grossir perpendiculairement à l'axe longitudinal sans se raccourcir en conséquence le long de l'axe longitudinal et inversement.

2. Corps protéique (forisome) selon la revendication 1,
**caractérisé par**
une longueur d'environ 1 µm à environ 40 µm et, perpendiculairement à cela, ayant un diamètre d'environ 1 µm à environ 10 µm.

3. Corps protéique (forisome) selon la revendication 1 ou 2,
**caractérisé en ce qu'**
il comporte une première protéine (P1) d'un poids moléculaire de l'ordre d'environ 55 à 65 kDa et une deuxième protéine (P2) d'un poids moléculaire de l'ordre de 53 à 63 kDa.

4. Corps protéique (forisome) selon la revendication 3,
**caractérisé en ce qu'**
à partir des protéines on peut identifier les peptides suivants en cas de digestion tryptique : et la protéine P1 contient par ailleurs les fragments suivants :

5. Corps protéique (forisome) selon l'une quelconque des revendications précédentes,
**caractérisé par**
une contractilité le long de l'axe longitudinal pouvant atteindre 30 % environ avec expansion simultanée d'environ 100 % perpendiculairement à celui-ci.

6. Protéine P1 ayant un poids moléculaire de l'ordre d'environ 55 à 65 kDa, comme définie dans la revendication 3 ou fragment de celle-ci, contenant la séquence suivante ou constitué(e) de la séquence suivante (lu de la terminaison N à la terminaison C) : et/ou contenant la séquence suivante ou constitué(e) de la séquence suivante (lu de la terminaison N à la terminaison C) :

7. Protéine P2 ayant un poids moléculaire de l'ordre d'environ 53 à 63 kDa, comme définie dans la revendication 3 ou un fragment de celle-ci contenant la séquence suivante ou constituée de la séquence suivante (lu de la terminaison N à la terminaison C) : et/ou contenant la séquence suivante ou constitué(e) de la séquence suivante (lu de la terminaison N à la terminaison C) :

8. Protéine P1 ayant un poids moléculaire de l'ordre d'environ 55 à 65 kDa, comme définie dans la revendication 3 ou fragment de celle-ci contenant les séquences suivantes ou constitué(e) des séquences suivantes (lu de la terminaison N à la terminaison C) :

9. Protéine P1 ou fragment de celle-ci selon la revendication 8, contenant en outre au moins l'une des séquences d'acides aminés suivantes : et

10. Procédé d'isolement de corps protéiques (forisomes) selon l'une quelconque des revendications 1 à 5,
**caractérisé par**
les étapes suivantes :
(a) obtention du phloème d'une plante de la famille des fabacées ;
(b) ouverture des cellules du phloème ;
(c) filtration d'une suspension des cellules ouvertes et
(d) séparation des forisomes des autres constituants de la suspension à l'aide d'une centrifugation en gradient.

11. Procédé selon la revendication 10,
**caractérisé en ce que**
la centrifugation en gradient est réalisée avec une solution de Nycodenz, le milieu de la suspension selon l'étape (c) contenant du KCl dans un tampon approprié.

12. Procédé selon la revendication 10 ou 11,
**caractérisé en ce que**
la plante appartient à la famille des fabacées Vicia faba.

13. Utilisation d'un ou plusieurs corps protéiques (forisomes) selon l'une quelconque des revendications 1 à 5 en tant qu'élément(s) contractif(s) dans une micropincette ou dans un élément de détection des modifications du pH ou des modifications de la concentration en ions calcium.

14. Procédé d'utilisation d'une micropincette,
**caractérisé en ce qu'**
un forisome de taille appropriée selon l'une quelconque des revendications 1 à 5 est disposé de manière stable entre deux branches à ressort et ensuite la concentration d'ions calcium dans l'environnement du forisome est augmentée de nettement en dessous de 30 nM à nettement au-dessus de 30 nM.

15. Procédé d'utilisation d'un élément d'affichage d'une modification des ions calcium de nettement en dessous 30 nM à nettement au-dessus de 30 nM ou d'une modification du pH de nettement en dessous de 9,4 à nettement au-dessus de 10,0 dans un milieu,
**caractérisé en ce qu'**
un forisome de taille appropriée selon l'une quelconque des revendications 1 à 6 est disposé de manière stable entre deux branches à ressort dont les pointes peuvent se toucher lors d'une contraction du forisome, les branches à ressort et leurs pointes étant configurées de manière à ce qu'un circuit fermé soit créé lors du contact indiqué et que le courant passe, le passage du courant ou l'interruption du circuit étant détecté(e) en tant que signal du fait que la concentration en ions calcium augmente nettement au-dessus de 30 nM, ou inversement respectivement que le pH augmente au-dessus de 10,0 ou descende en dessous de 9,4.

## Claims

1. Protein body (forisome), **characterised by:**
- its producibility from Fabaceae and
- a reversible, anisotropic contractability having the capacity, with an increase in the surrounding calcium ion concentration above a threshold value of approx. 30 nm, to become thicker in the directions perpendicular to the longitudinal axis and shorter along the longitudinal axis and vice versa, and having the capacity, with an increase in the surrounding pH value to above approx. 9.5, to become thicker perpendicular to the longitudinal axis without being correspondingly shortened along the longitudinal axis and vice versa.

2. Protein body (forisome) according to claim 1, **characterised by** a length of approx. 1 µm up to approx. 40 µm and perpendicular thereto with a diameter of approx. 1 µm up to approx. 10 µm.

3. Protein body (forisome) according to claim 1 or 2, **characterised in that** it comprises a first protein (P1) with a molecular weight in the range of approx. 55 - 65 kDa and a second protein (P2) with a molecular weight in the range of 53 - 63 kDa.

4. Protein body (forisome) according to claim 3, **characterised in that** the following peptides can be detected from the proteins during tryptic digestion: and **in that** the protein P1 contains furthermore the following fragments:

5. Protein body (forisome) according to one of the preceding claims, **characterised by** a contractability along the longitudinal axis of up to approx. 30% with simultaneous expansion perpendicular thereto by approx. 100%.

6. Protein P1 with a molecular weight in the range of approx. 55 - 65 kDa as defined in claim 3 or fragment thereof, containing or comprising the following sequence (read from N terminal to C terminal) : and/or containing or comprising the following sequence (read from N terminal to C terminal):

7. Protein P2 with a molecular weight in the range of approx. 53 - 63 kDa as defined in claim 3 or fragment thereof, containing or comprising the following sequence (read from N terminal to C terminal): and/or containing or comprising the following sequence (read from N terminal to C terminal):

8. Protein P1 with a molecular weight in the range of approx. 55 - 65 kDa as defined in claim 3 or fragment thereof, containing or comprising the following sequences (read from N terminal to C terminal):

9. Protein P1 or fragment thereof according to claim 8, containing furthermore at least one of the following amino acid sequences: and

10. Method for isolating protein bodies (forisomes) according to one of the claims 1 to 5, **characterised by** the following steps:
(a) obtaining the phloem of a plant from the Fabaceae family;
(b) breaking down the cells of the phloem;
(c) filtering a suspension of broken-down cells; and
(d) separating the forisomes from the remaining components of the suspension with the help of gradient centrifugation.

11. Method according to claim 10, **characterised in that** the gradient centrifugation is effected with a Nycodenz solution, the medium of the suspension according to step (c) containing KCl in a suitable buffer.

12. Method according to claim 10 or 11, **characterised in that** the plant from the Fabaceae family is Vicia faba.

13. Use of one or more protein bodies (forisomes) according to one of the claims 1 to 5 as contractive element(s) in a micro-pincette or in a detection element for pH value changes or changes in calcium ion concentration.

14. Method for operating a micro-pincette, **characterised in that** a forisome of a suitable size according to one of the claims 1 to 5 is disposed in a stable manner between two spring arms and subsequently the concentration of calcium ions in the surroundings of the forisome is increased from significantly below 30 nm to significantly above 30 nm.

15. Method for operating a display element for a change in calcium ions from significantly below 30 nm to significantly above 30 nm or for a change in pH value from below pH 9.4 to above pH 10.0 in a medium, **characterised in that** a forisome of a suitable size according to one of the claims 1 to 6 is disposed in a stable manner between two spring arms, the tips of which can contact each other during a contraction of the forisome, the spring arms and their tips being configured such that a circuit is closed during the mentioned contact and current flows, the flow of current or the interruption of the circuit being detected as a signal that the calcium ion concentration is increasing to significantly above 30 nm or vice versa or that the pH value is increasing to above pH 10.0 or dropping below 9.4.


### SEQUENCE LISTING

<110> Fraunhofer-Gesellschaft zur Förderung der angewandten Forschung e.V.
<120> Forisomen, Verfahren zu ihrer Isolierung und ihre Verwendung als molekulare Arbeitsmaschinen
<130> 11154e
<160> 5
<170> PatentIn version 3.2
<210> 1
   <211> 10
   <212> PRT
   <213> vicia faba
<220>
   <221> PEPTIDE
   <222> (1)..(10)
   <223> Glx in Positionen 2 und 6 ist Gln
   Glx in Position 7 ist Glu
   Asx in Position 3 ist Asp
   Asn in Position 4 ist Asn
<400> 1
   Leu Glx Asx Asx Pro Glx Glx Val Ile Lys
   1               5                   10
<210> 2
   <211> 8
   <212> PRT
   <213> vicia faba
<220>
   <221> PEPTIDE
   <222> (1)..(8)
   <223> Glx in Position 1 ist Glu
   Asx in Position 4 ist Asp
<400> . 2
<210> 3
   <211> 5
   <212> PRT
   <213> vicia faba
<220>
   <221> PEPTIDE
   <222> (1)..(5)
<400> 3
   Glu Val Thr Ser Val
   1 5
<210> 4
   <211> 9
   <212> PRT
   <213> vicia faba
<220>
   <221> PEPTIDE
   <222> (1)..(9)
<400> 4
   Val Met Glu Val Ser Trp His Tyr Lys
   1 5
<210> 5
   <211> 4
   <212> PRT
   <213> vicia faba
<220>
   <221> PEPTIDE
   <222> (1)..(4)
<400> 5
   Ala Thr Asp Pro
   1